(19) Europäisches Patentamt
European Patent Office
Office européen des brevets

(11) Veröffentlichungsnummer : **0 173 827**
**B1**

(12) **EUROPÄISCHE PATENTSCHRIFT**

(45) Veröffentlichungstag der Patentschrift : **25.05.88**

(51) Int. Cl.[4] : **C 07 C 29/86**, C 07 C 31/20

(21) Anmeldenummer : **85108773.4**

(22) Anmeldetag : **13.07.85**

(54) **Verfahren zur Aufarbeitung substituierter, wenig wasserlöslicher 1,3-Diole.**

(30) Priorität : **06.09.84 DE 3432719**

(43) Veröffentlichungstag der Anmeldung : **12.03.86 Patentblatt 86/11**

(45) Bekanntmachung des Hinweises auf die Patenterteilung : **25.05.88 Patentblatt 88/21**

(84) Benannte Vertragsstaaten : **DE FR GB IT**

(56) Entgegenhaltungen : **Keine Entgegenhaltungen**

(73) Patentinhaber : **HÜLS AKTIENGESELLSCHAFT
- RSP Patente / PB 15 - Postfach 13 20
D-4370 Marl 1 (DE)**

(72) Erfinder : **Kleine Homann, Walter, Dr.
Buchenallee 14
D-4408 Dülmen (DE)**

**EP 0 173 827 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Substituierte 1,3-Diole der allgemeinen Formel

$$\underset{OH}{R-CH}-CR_2-\underset{OH}{CH}-R$$

mit R = Alkyl und/oder Wasserstoff sind leicht durch Aldolisationsreaktionen mit nachfolgender Hydrierung zugänglich :

$$RCHO + CR_2H-CRO \longrightarrow R-\underset{OH}{CH}-CR_2-\underset{R}{C}{=}O \xrightarrow{H_2} R-\underset{OH}{CH}-CR_2-CHR(OH)$$

Als Nebenprodukt treten häufig Ester auf, die bei der Destillation zum einen zu Produktverlusten führen, zum anderen einen überproportionalen Trennaufwand erfordern.

Zwar sind Ester prinzipiell verseifbar, jedoch ergeben sich bei einem derartig behandelten Rohprodukt neue Probleme bei der Destillation durch partielle Produktzersetzungen. Zur Vermeidung dieser Probleme wird in der JP-PS 69/10767 der Einsatz von Extraktionsmitteln wie Di-n-butylether im Falle des gut wasserlöslichen 2,2-Dimethylpropandiols-1,3 (NPG) empfohlen. Ebenfalls beim NPG wird eine Vorbehandlung mittels eines Dünnschichtverdampfers angeraten, um Salze und Höhersieder, die während der Synthese anfallen, abzutrennen. Gemäß der US-PS 2 865 819 wird durch Zugabe von Wasser und Tetralin NPG von den Salzen der Verseifung destillativ in mehreren Stufen abgetrennt. Auch der Einsatz von Ionenaustauschern zur Entfernung von im Rohprodukt enthaltenen Salzen wird angegeben, DE-PS 967 552.

Die Nachteile dieser Verfahren sind u. a. darin zu sehen, daß sie Hilfsstoffe benötigen, die energieintensiv entfernt werden müssen und/oder teuer vom Verbrauch her sind bzw. Wertstoffverluste unberücksichtigt lassen.

Die sich hieraus ergebende Aufgabe wird erfindungsgemäß entsprechend den Angaben der Patentansprüche gelöst.

Substituierte, wenig wasserlösliche 1,3-Diole lassen sich überraschenderweise problemlos aufarbeiten, wenn das Rohprodukt zunächst entsprechend der Verseifungszahl bzw. einer größeren Menge bei erhöhter Temperatur, im allgemeinen von 80 bis 130 °C, mit basischen Zusätzen behandelt wird ; um danach mit 5 bis 30 %, vorzugsweise 10 bis 20 % Wasser versetzt zu werden. Die wäßrige Phase trennt man mechanisch ab. Nach dem Separieren wird die organische Phase nach Zugabe von 5 bis 30 % Wasser auf einen pH-Wert von 8,0 bis 10,5 vorzugsweise 8,5 bis 9,5, eingestellt und die wäßrige Phase erneut entfernt. In der nachfolgenden Destillation wird nach Abtrennung der einwertigen Alkohole wiederum ein pH-Wert von 8,0 bis 10,5, vorzugsweise 8,5 bis 9,5 eingestellt. Eine geringere als der Verseifungszahl entsprechende Menge führt lediglich zu einer geringfügigen Reduzierung obiger Probleme. Als basische Zusätze können u. a. Alkali- und Erdalkalihydroxide sowie Ammoniak oder Amine verwendet werden.

Es ist angezeigt, die Reaktion bei möglichst hoher Temperatur durchzuführen, im allgemeinen 80 bis 130 °C, vorzugsweise etwa 10 °C unterhalb des Siedepunktes des im Rohaustrag enthaltenen Niedrigsieders, z. B. monofunktionelle Alkohole, die bei der Hydrierung aus den Carbonylverbindungen entstehen. Unter Inkaufnahme verlängerter Reaktionszeiten ist jedoch die Behandlung auch bei wesentlich niedrigeren Temperaturen möglich. Wird die Verseifung im Sidebereich des Niedrigsieders vorgenommen, so ist eine Bearbeitung unter Rückfluß angezeigt.

Die Konzentration des basischen Zusatzes ist nicht beliebig zu reduzieren, da sich andernfalls die Reaktionszeit unnötig verzögert. Als sinnvoll haben sich Konzentrationen der Lösungen von beispielsweise 10 bis 50 % erwiesen.

Analog verhält es sich bei der « Neutralisation » auf den obigen pH-Bereich. Zur pH-Wert-Einstellung eignen sich beispielsweise anorganische Säuren wie Schwefelsäure oder Phosphorsäure und Ionenaustauscher.

Nach der Verseifung, jedoch vor der pH-Wert-Einstellung ist der Zusatz von 5 bis 30 % Wasser wünschenswert, um letzte problemloser gestalten zu können. Während sich je nach Laugenkonzentration bei geringerem Wasserzusatz häufig keine ausreichende Phasentrennung ergibt, ist bei größeren Wassermengen der Wertstoffverlust unerwünscht hoch. Zur Minimierung von Produktverlusten bei der Separierung mittels geeigneter Trennvorrichtungen ist es ratsam, erst nach entsprechender Kühlung im allgemeinen auf 1 bis 30 °C die Separierungen vorzunehmen.

Neben einer diskontinuierlichen Realisierung führt auch die kontinuierliche Methode zu dem gewünschten Ergebnis.

Beispiele

a) 500 g eines rohen 2,2,4-Trimethylpentandiols (TMPD) mit einer Verseifungszahl von 7,5 wurden mit 6,5 g einer 50 %igen NaOH-Lösung bei 90 °C gerührt und danach mit 100 g Wasser auf 20 °C abgekühlt. Die organische Phase wurde nach Zugabe von 100 g Wasser mittels Dosierung von 10 %iger Schwefelsäure auf einen pH-Wert von 9 eingestellt. Nach dem Abtrennen des in der organischen Phase enthaltenen Isobutanols bei Normaldruck, wurde das TMPD unter portionsweiser Zugabe von konz. Schwefelsäure derart, daß im Sumpf ein pH-Wert von ca. 9 eingehalten wurde, im Wakuum fraktioniert.

Bezogen auf im Rohprodukt enthaltenes TMPD wurden 97 % an TMPD mit einer Reinheit von 99,2 % gewonnen.

Vergleichbare Werte wurden beim Einsatz von Kaliumhydroxid, Calciumhydroxid und Trioctylamin als Basen und Phosphorsäure sowie dem Ionenaustauscher Lewatit SPC 108 zur « Neutralisation » erzielt. Bei einem Bereich an TMPD-Ausbeuten von 96 bis 99 % konnte Reinprodukt mit 98,8 bis 99,8 % isoliert werden.

b) 3 kg eines rohen 2-Ethylhexandiols-1,3 (EHD) mit einer Verseifungszahl von 8,5 wurden mit 80 g einer 25 %igen NaOH-Lösung bei 80 °C wie im Beispiel a) aufgearbeitet.

Bezogen auf im Rohprodukt enthaltenes EHD wurden 99 % an EHD mit einer Reinheit von 99,3 % gewonnen.

c) 3 kg eines rohen 2-n-Propylheptandiol-1,3 (PHD) mit einer Verseifungszahl von 12,6 wurden mit 300 g einer 10 %igen NaOH-Lösung bei 100 °C wie im Beispiel a) aufgearbeitet.

Bezogen auf im Rohprodukt enthaltenes PHD wurden 98 % an PHD mit einer Reinheit von 98,9 % gewonnen.

## Patentansprüche

1. Verfahren zur Aufarbeitung substituierter, wenig wasserlöslicher 1,3-Diole, dadurch gekennzeichnet, daß man

a) das durch Aldolisierung und nachfolgende Hydrierung erhaltene Rohprodukt bei erhöhter Temperatur entsprechend der Verseifungszahl bzw. einer größeren Menge mit basischen Zusätzen behandelt,

b) anschließend mit 5 bis 30 % Wasser versetzt und gegebenenfalls auf 1 bis 30 °C abkühlt,

c) die wäßrige Phase mechanisch abtrennt,

d) die organische Phase nach dem Separieren nach Zugabe von 5 bis 30 % Wasser auf einen pH-Wert von 8,0 bis 10,5 einstellt,

e) die wäßrige Phase erneut entfernt,

f) anschließend den einwertigen Alkohol destillativ abtrennt,

g) den pH-Wert wiederum auf 8,0 bis 10,5 einstellt und

h) das 1,3-Diol durch Fraktionierung gewinnt.

2. Verfahren nach Anspruch 1, dadurch gekennzeichnet, daß man in den Stufen d) und/oder g) einen pH-Wert von 8,5 bis 9,5 einstellt.

## Claims

1. A process for working up a substituted, sparingly water-soluble 1,3-diol, characterized in that

a) the crude product obtained through aldolization and subsequent hydrogenation is treated at elevated temperature with a basic additive in an amount corresponding to the hydrolysis number or in a larger amount,

b) 5 to 30 % of water is subsequently added and the mixture is cooled, if desired, to 1 to 30 °C,

c) the aqueous phase is separated off mechanically,

d) after the separation the organic phase is adjusted to a pH of 8,0 to 10,5 after addition of 5 to 30 % of water,

e) the aqueous phase is again removed,

f) the monohydric alcohol is subsequently removed by distillation,

g) the pH is again adjusted to 8,0 to 10,5, and

h) the 1,3-diol is obtained by fractionation.

2. A process according to claim 1, characterized in that the pH in steps d) and/or g) is adjusted to 8,5 to 9,5.

## Revendications

1. Procédé de retraitement de 1,3-diols substitués, peu solubles dans l'eau, caractérisé par le fait que

a) on traite par des additifs basiques le produit brut obtenu par aldolisation et hydrogénation

subséquente à une température élevée correspondant à l'indice de saponification ou à une plus grande quantité,

b) on ajoute ensuite de 5 à 30 % d'eau et on refroidit éventuellement à une température de 1 à 30 °C,

c) on sépare mécaniquement la phase aqueuse,

d) on ajuste le pH de la phase organique, à une valeur située de 8,0 à 10,5 après la séparation et l'addition de 5 à 30 % d'eau,

e) on élimine à nouveau la phase aqueuse,

f) on sépare ensuite le mono-alcool par distillation,

g) on porte à nouveau le pH à une valeur de 8,0 à 10,5, et

h) on obtient le 1,3-diol par fractionnement.

2. Procédé selon la revendication 1, caractérisé par le fait que le pH est ajusté à une valeur de 8,5 à 9,5 dans les étapes d) et/ou g).